# EUROPEAN PATENT SPECIFICATION

(11) **EP 2 749 270 B1**
(45) Date of publication and mention of the grant of the patent: **17.01.2018**
(21) Application number: 13005858.9
(22) Date of filing: 17.12.2013
(51) Int. Cl.: A61K 9/16, A61K 9/20, A61K 9/50, A61K 31/223

(54) **Racecadotril and pharmaceutical compositions thereof**
Racecadotril und pharmazeutische Zusammensetzungen daraus
Racécadotril et ses compositions pharmaceutiques

(30) Priority: 26.12.2012 TR 201215298
(43) Date of publication of application: 02.07.2014
(73) Proprietor: ILKO Ilaç Sanayi ve Ticaret A.S., Sancaktepe/Istanbul (TR)
(72) Inventor: Öncel, Hatice, Sancaktepe, Istanbul (TR); Yildiz, Simay, Ankara (TR); Çapan, Yilmaz, Ankara (TR); Gurbetoglü, Gülistan Pelin, Ankara (TR); Doganay, Asuman Aybey, Ankara (TR); Bulut, Burcu, Ankara (TR); Pinarbasli, Onur, Ankara (TR); Sarraçoglu, Nagehan, Ankara (TR)
(74) Representative: Bulut, Pinar

(56) References cited:
- WO-A2-01/97801
- US-A1- 2009 186 084

## Description

### Technical field:

The present invention is related to pharmaceutical compositions comprising racecadotril whose taste is masked by using wet granulation method, for the treatment of diarrhea with racecadotril whose taste is masked.

### Prior Art:

Racecadotril is an active substance used in diarrhoea treatment (I). It is first described in US4513009. Racecadotril is also known as acetorphane. The pharmaceutical products comprising racecadotril are present in the market with the trade names Hidrasec, Tiorfan, Tiorfix.

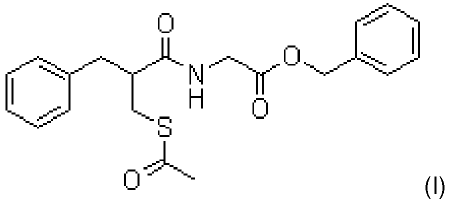

Its capsule form, tablet form and sachet form (used for children) present in the market comprises 100mg, 175mg and 10mg or 30mg Racecadotril respectively.

EP1294372 (Bioproject) discloses the dry powder formulation of racecadotril which is preferred for pediatric usages. The characteristic of the formulation is that, the racecadotril granulate prepared with a suitable carrier is coated and mixed with a sweetener. Acrylate and methacrylate polymers, especially Eudragit NE 30D are stated as coating agents. According to the procedure, first, racecadotril granulate is prepared with a little sugar, it is coated with a coating agent; the coated granule is mixed with the remaining sugar, aerosil and sweetener and then filled into sachet.

EP2018158 (Bioproject) is related to Racecadotril tablet formulation, WO 2001/097803 (Glaxosmithkline) is related to its capsule formulation and EP 2462922 (Bioproject) is related to its suspension formulation for usage in children.

As it is known, in drug administration, mostly, the suspension form is preferred in terms of convenience. However, as racecadotril is a hydrophobic substance, difficulties are present in its preparation in suspension form. Additionally, in dosage forms such as dry powder formulations, there are problems such as dose uniformity. Therefore, dispersible tablet forms are preferred which may be administered to the children easily, have an easier production and also provide a homogenous dosage distribution.

Dispersible tablets, in other words, orally disintegrating tablets disperse in water quickly. They are characterized in that, their dispersion time is less than 1 minute, preferably it is 40 seconds or less than 40 seconds. As they can disperse the mouth, swallowing them with water is not needed. In addition to these, the taste and the hardness of the tablets should be acceptable.

### Description of the Invention:

According to the invention, it is provided a dispersible tablet comprising racecadotril, whose taste is masked, characterized in that the taste of the active substance racecadotril is masked by coating with ethyl acrylate-methyl methacrylate copolymer, by using wet granulation method without being mixed with any excipient, wherein the amount of ethyl acrylate-methyl methacrylate copolymer is from 1 to 10% by weight of the amount of racecadotril.

The formulation is prepared wet granulation technique and compressed as dispersible tablet (oral disintegrating tablet). During the wet granulation process, the racecadotril compound is directly subjected to wet granulation without being mixed with any excipient or carrier. Thus, the bitter taste of the racecadotril compound is masked. The wet granulation of the racecadotril compound solely with a taste masking agent is a new process for state of art.

Cellulose polymers such as hydroxypropylmethyl cellulose (HPMC), hydroxyethylcellulose, hydroxymethylcellulose, hydroxypropylcellulose, hydroxyethylmethyl cellulose, sodium carboxymethylcellulose, ethyl cellulose and methyl cellulose and acrylic acid polymers or copolymers may be used for masking the taste with wet granulation process. Ethyl acrylate - methyl methacrylate copolymer, dimethyl aminoethyl methacrylate copolymer (Eudragit E 100, Eudragit E 12,5 and Eudragit E PO) with ammonium methacrylate copolymer (Eudragit RL and Eudragit RS) may be used as acrylate polymer. For wet granulation, ethyl acrylate - methyl methacrylate copolymer that can be dispersed in water is chosen. The ratio of ethyl acrylate to methyl methacrylate in copolymer is about 2:1. This polymer, which is in the form of aqueous dispersion of ethyl acrylate and methyl methacrylate based neutral copolymer, is present in the market with the trade name of Eudragit NE 30 D. The solid matter amount of the wet dispersion is 30 %. This polymer is present in European pharmacopeia as Polyacrylat Dispersion 30 Percent monograph and present in US/NF as Ethyl Acrylate and Methyl Methacrylate Copolymer Dispersion.

EP1294372 (Bioproject) use ethyl acrylate-methyl methacrylate copolymer 15% by weight of the amount of racecadotril in sachet form however in dispersible tablet formulation methyl acrylate-methyl methacrylate copolymer ratio is in the range of 1 to 10 % by weight of the amount of racecadotril to achieve the desired dissolution profile and taste masking.

Like racecadotril tablets, the dispersible tablets comprise 5mg to 175 mg racecadotril. The preferred unit dosage is 30mg.

Racecadotril, whose taste is masked by being subjected to wet granulation, is used as intermediate product in the preparation of pharmaceutical compositions such as tablet, dispersible tablet and suspension.

Racecadotril, whose taste is masked by wet granulation method for dispersible tablet formulation, is transformed into tablet by being mixed with the excipients mentioned below.

Polyols such as mannitol, sorbitol, maltitol and xylitol may be used as diluents and viscosity agents. Mannitol known with the trade name Pearlitol is preffered. In the dispersible tablet, DC mannitol may be used with an amount of 30-70%. It is preferably used in amount of 50% approximately. In prior art, the usage of sugar as diluent for Racecadotril granulate is known (EP1294372). As a new feature, the newly developed Racecadotril dispersible tablet comprises mannitol.

Although there are various compounds used as disintegrant in the tablets, Kollidon CL, which is known as super disintegrant is preferred as disintegrant. Cross linked povidone (cross linked polyvinylpyrolidone) that is known as Kollidon CL, is also known as crospovidone and water-insoluble polyvinylpyrolidone. In the dispersible tablet, the amount of crospovidon used is 5-25 %.

Additionally, pregelatinized starch having binding, disintegrating and lubricant features is also used in the tablets. In the dispersible tablet, the amount of pregelatinized starch used is 5-30 %.

Various stearate compounds, stearic acid, vegetable oils such as hydrogenated castor oil may be used as lubricant in the tablets. In order to prevent the metallic taste left in mouth due to the alkaline structure of magnesium stearate and in order to increase the patient compliance, sodium stearyl fumarate is used as lubricant in dispersible tablets.

Various sweeteners such as saccharine, sodium cyclamate, acesulfam potassium, aspartame, sucralose may be used as sweeteners. Aspartam-acesulfam potassium mixture is chosen as the sweetener system. In order to give a suitable flavor to the dispersible tablet, approximately 6 % sweetener mixture by weight is used. The ratio of aspartame to acesulfam potassium is 5:2 parts by weight.

Strawberry aroma is chosen among fruit aromas such as banana, strawberry, raspberry, peach.

Examples below are for the explanation of the invention, but they do not restrict it.

### Example 1

Racecadotril 30mg Dispersible Tablet is prepared according to the unit formula below:

| **Ingredients** | **(mg)** | **(%)** |
|---|---|---|
| Racecadotril | 30,0 | 12,50 |
| Eudragit NE 30D | 0,6 | 0,25 |
| Aspartam | 10,0 | 4,17 |
| Kollidon CL | 30,0 | 12,50 |
| Mannitol | 125,6 | 52,33 |
| Acesulfame K | 4,0 | 1,67 |
| Pregelatinized starch | 26,0 | 10,83 |
| Strawberry flavor | 9,0 | 3,75 |
| Sodium stearyl fumarate | 4,8 | 2,00 |
| **Total** | **240,0** | **100,00** |

Racecadotril is sieved through a 0.8 mm sieve and prepared for wet granulation by adding Eudragit NE-30D thereon prepared with distilled water. After the sieving and drying of wet granulates, they are first mixed with aspartam, Kollidon CL, mannitol, acesulfam K, Prejelatinized starch and strawberry aroma and then, with sodium stearyl fumarate and compressed as tablets.

### Example 2

Racecadotril 30mg Dispers Tablet is prepared according to the unit formula below:

| **Ingredients** | **(mg)** | **(%)** |
|---|---|---|
| Racecadotril | 30,0 | 12,50 |
| HPMC | 2,4 | 1,00 |
| Aspartam | 10,0 | 4,17 |
| Kollidon CL | 30,0 | 12,50 |
| Mannitol | 123,8 | 51,58 |
| Acesulfame K | 4,0 | 1,67 |
| Pregelatinized starch | 26,0 | 10,83 |
| Strawberry flavor | 9,0 | 3,75 |
| Sodium stearyl fumarate | 4,8 | 2,00 |
| **Total** | **240,0** | **100,00** |

Racecadotril is sieved through a 0.8 mm sieve and prepared for wet granulation by adding HPMC solution thereon prepared with distilled water. After the sieving and drying of wet granulates, they are first mixed with aspartam, Kollidon CL, mannitol, acesulfam K, prejelatinized starch and strawberry aroma and then, with sodium stearyl fumarate and compressed as tablets.

As the taste masking efficiency of the water soluble polymer is less than the water insoluble polymer, the amount of HPMC polymer used in taste masking is more than the amount of Eudragit NE 30 D.

## Claims

1. A dispersible tablet comprising Racecadotril whose taste is masked, **characterized in that**, the taste of the active substance, racecadotril is masked by coating with ethyl acrylate-methyl methacrylate copolymer, by using wet granulation method without being mixed with any excipient wherein the amount of ethyl acrylate-methyl methacrylate copolymer is from 1 to 10% by weight of the amount of racecadotril.

2. A dispersible tablet according to the claim 1, wherein it comprises by weight 30-70% of mannitol, 5-25% of crospovidone, 5-30% of pregelatinized starch and 0.5-2% of sodium stearyl fumarate according to the weight of tablet.

3. A dispersible tablet according to claim 2, wherein it comprises aspartam and acesulfam potassium as sweetener.

4. A dispersible tablet according to claim 3, wherein the ratio of aspartame to acesulfam potassium is 3:1 to 2:1 by weight.

5. A dispersible tablet according to Claim 1, wherein the amount of Racecadotril is 5 to 175 mg in tablet.

6. A dispersible tablet according to claim 5, wherein it comprises 30mg of Racecadotril.

## Patentansprüche

1. Eine zerstreubare Tablette bestehend aus racecadotril, dessen Geschmack verdeckt ist, **dadurch gekennzeichnet, daß** der Geschmack von aktiver Substanz, racecadotril verdeckt ist durch Bedeckung mit Ethylacrylat-Methylmethacrylat Kopolymer, durch Verwendung von nasse Granulationsmethode ohne Einmischung mit irgendwelcher Arzneistoffträger, wo die Menge von Ethylacrylat-Methylmethacrylat Kopolymer von 1 bis 10% im Gewicht aus racecadotril ist.

2. Eine zerstreubare Tablette nach Anspruch 1, wo sie beinhaltet im Gewicht 30-70% mannitol, 5-25% crospovidone, 5-30% vorgelatinisierte Stärke, und 0.5-2% Natriumstearyl- fumarat je nach dem Gewicht der Tablette.

3. Eine zerstreubare Tablette nach Anspruch 2, wo sie beinhaltet aspartame und acesulfam- potassium als Süßstoff.

4. Eine zerstreubare Tablette nach Anspruch 3, wo das Verhältnis von aspartame gegen acesulfampotassium 3:1 bis 2:1 im Gewicht ist.

5. Eine zerstreubare Tablette nach Anspruch 1, wo die Menge von racecadotril von 5 bis 175 mg in Tablette ist.

6. Eine zerstreubare Tablette nach Anspruch 5, wo sie beinhaltet 30 mg racecadotril.

## Revendications

1. Comprimé dispersible comprenant du racecadotril dont le goût est masqué, **caractérisé en ce que**, le goût de la substance active, le racecadotril est masqué par revêtement avec un copolymère acrylate d'éthyle-méthacrylate de méthyle, en utilisant un procédé de granulation humide sans être mélangé avec un quelconque excipient dans lequel la quantité d'éthyle acrylate-méthacrylate de méthyle est de 1 à 10% en poids de la quantité de racecadotril.

2. Comprimé dispersible selon la revendication 1, dans lequel il comprend en poids 30 à 70% de mannitol, 5 à 25% de crospovidone, 5 à 30% d'amidon prégélatinisé et 0,5 à 2% de stéaryl fumarate de sodium selon le poids du comprimé.

3. Comprimé dispersible selon la revendication 2, dans lequel il comprend de l'aspartame et de l'acésulfame de potassium comme édulcorant.

4. Comprimé dispersible selon la revendication 3, dans lequel le rapport entre l'aspartame et l'acésulfamate de potassium est de 3:1 à 2:1 en poids.

5. Comprimé dispersible selon la revendication 1, dans lequel la quantité de Racecadotril est de 5 à 175 mg en comprimé.

6. Comprimé dispersible selon la revendication 5, dans laquelle elle comprend 30 mg de Racecadotril.
